# EUROPEAN PATENT APPLICATION

(11) **EP 4 356 825 A1**
(43) Date of publication of application: **24.04.2024**
(21) Application number: 22213877.8
(22) Date of filing: 15.12.2022
(51) Int. Cl.: A61B 5/01

(54) **SYSTEMS AND METHODS FOR DETECTING ORAL CONDITIONS USING AN ORAL CARE DEVICE**

(30) Priority: 22.10.2022 US 202263418532 P
(71) Applicant: Koninklijke Philips N.V., 5656 AG Eindhoven (NL)
(72) Inventor: GOTTENBOS, Bart, Eindhoven (NL); COOMBS, Jim, Eindhoven (NL)
(74) Representative: Philips Intellectual Property & Standards

(57) **Abstract**

The present disclosure is directed to systems and methods for detecting an oral condition of a subject during an oral treatment procedure. More specifically, the present disclosure is directed to systems and methods that employ an oral care device configured to induce thermal changes over a target region within a subject's oral cavity during an oral treatment procedure, and then thermally detect one or more oral conditions without stopping the oral treatment procedure. In embodiments, the oral care system includes an oral care device comprising a temperature change element and a temperature sensing element, both disposed at least partially within a head portion of the oral care device that is configured to be received in the subject's oral cavity.

## Description

### Field of the Disclosure

The present disclosure relates generally to systems and methods for detecting an oral condition of a subject using an oral care device, for example during an oral treatment operation, and more specifically to systems and methods that employ an oral care or surgical device configured to thermally detect one or more oral conditions while performing an oral treatment operation or dental surgery.

### Background

The field of electric devices used for oral care along with new features and functionalities is rapidly expanding. Advancements in this field have resulted in oral care devices, such as electric toothbrushes, that are capable of tracking a user's time using the device, how much pressure the user applies using the device, which regions of the mouth the user cleans, and so on. Separately, advancements have been made in medical devices that can detect various oral conditions, such as the presence of dental plaque. Despite these advancements, medical devices for detecting various oral conditions continue to require additional steps after a treatment procedure, require application of additional formulations to the teeth, require expensive lasers, and/or are simply unreliable. Furthermore, these medical devices are unable to provide cleaning and/or treatment feedback while the treatment procedure is still ongoing such that the user has the opportunity to immediately address the dental concern.

### Summary of the Disclosure

In accordance with the present disclosure, Applicants have recognized and appreciated that many oral conditions can be detected by inducing a temperature change at a target site and measuring the temperature gradient over time due to the differences in thermal properties of the affected and unaffected tissues. For example, the presence and/or severity of a plaque build-up can be detected by inducing a temperature change within a subject's oral cavity and measuring the temperature gradient of the affected areas (i.e., where the plaque is present) versus the unaffected area (i.e., where there is little to no plaque). In further examples, the presence and/or severity of other dental or oral conditions may be detected, as discussed in detail below.

According to an embodiment of the present disclosure, an oral care system is provided. The oral care system comprises: an oral care device including (i) a head portion adapted to be received in an oral cavity of a subject for an oral treatment procedure, and (ii) a body portion operatively connected to the head portion; wherein the head portion of the oral care device comprises: a temperature change element configured to induce a change in temperature of a target region of the oral cavity; and a temperature sensing element configured to detect variations in temperature over at least a section of the target region of the oral cavity.

In an aspect, the head portion of the oral care device further comprises a treatment feature configured to facilitate the oral treatment procedure, and the temperature change element is configured to induce a change in temperature of a target region of the oral cavity during the oral treatment procedure in proximity thereof.

In an aspect, the treatment feature comprises at least one of bristles, pillars, and woven textiles.

In an aspect, the temperature change element is configured to induce a change in temperature of the target region of the oral cavity during the oral treatment procedure by at least one of: (i) directing an airstream to the target region of the subject's oral cavity; (ii) directing a liquid stream to the target region of the subject's oral cavity; directing electromagnetic radiation to the target region of the subject's oral cavity; and (iv) heating and/or cooling the temperature change element and then contacting at least a portion of the target region with the temperature change element.

In an aspect, the oral care system is configured to determine an oral condition within the oral cavity of the subject based on the detected variations in temperature induced by the temperature change element over at least the target region of the oral cavity.

In an aspect, the oral condition is at least one of the presence of dental plaque, dental caries, dental calculus, tooth decay, enamel defects, cavities, and/or soft tissue infection.

In an aspect, the temperature sensing element includes at least one of a thermal camera, a thermal radiation sensor or sensor array, and/or a contact temperature sensor.

In an aspect, the body portion of the oral care device comprises a user feedback mechanism configured to provide an alert indicating the existence of an oral condition within the oral cavity of the subj ect.

In an aspect, the oral care system further comprises one or more processors in communication with the oral care device and configured to: operate the temperature change element to induce a change in temperature of a target region of the oral cavity; operate the temperature sensing element to detect variations in temperature over at least a section of the target region of the oral cavity; determine, based on the detected variations in temperature over at least the target region of the oral cavity, one or more oral conditions; and generate an alert indicating the one or more oral conditions.

According to another embodiment of the present disclosure, a method of detecting an oral condition of a subject is provided. The method comprises: initiating an oral treatment procedure on the subject utilizing an oral care system comprising an oral care device, the oral care device including (i) a head portion adapted to be received in an oral cavity of the subject for the oral treatment procedure, and (ii) a body portion operatively connected to the head portion, wherein the head portion of the oral care device comprises (i) a temperature change element configured to induce a change in temperature of a target region of the oral cavity during the oral treatment procedure, and (ii) a temperature sensing element configured to detect variations in temperature over at least a section of the target region of the oral cavity; and while performing the oral treatment procedure: inducing a change in temperature of a target region of the oral cavity using the temperature change element of the oral care device; and detecting variations in temperature over at least the section of the target region of the oral cavity using the temperature sensing element of the oral care device.

In an aspect, the body portion of the oral care device comprises a user feedback mechanism configured to provide an alert indicating the existence of an oral condition within the oral cavity of the subject, and wherein the method further comprises: determining, based on the detected variations in temperature over at least the target region of the oral cavity, one or more oral conditions; and providing, via the feedback mechanism, an alert to a user of the oral care device indicating the existence of the one or more oral condition within the oral cavity of the subject.

In an aspect, the oral condition is at least one of the presence of dental plaque, dental caries, dental calculus, tooth decay, enamel defects, cavities, and/or soft tissue infection.

According to yet another embodiment of the present disclosure, an oral condition detection module configured to be attached to an oral care device is provided. The oral condition detection module comprises: a temperature change element configured to induce a change in temperature of a target region of a subject's oral cavity; and a temperature sensing element configured to detect variations in temperature over at least a section of the target region of the subject's oral cavity; wherein the oral condition detection module is configured to be attached to a head portion of the oral care device, the head portion and the oral condition detection module being configured to be received in the subject's oral cavity.

In an aspect, the temperature change element is configured to induce a change in temperature of the target region of the subject's oral cavity during the oral treatment procedure by at least one of: (i) directing an airstream to the target region of the subject's oral cavity; (ii) directing a liquid stream to the target region of the subject's oral cavity; directing electromagnetic radiation to the target region of the subject's oral cavity; and (iv) heating and/or cooling the temperature change element and then contacting at least a portion of the target region with the temperature change element.

In an aspect, the oral condition detection module further comprises a user feedback mechanism configured to provide an alert indicating the existence of an oral condition within the subject's oral cavity.

These and other aspects of the various embodiments will be apparent from and elucidated with reference to the embodiments described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a block diagram of an oral care system comprising an oral care device illustrated according to aspects of the present disclosure.
FIG. 2 is a drawing of an oral care device illustrated according to aspects of the present disclosure.
FIG. 3 is a drawing of a portion of an oral care device illustrated according to aspects of the present disclosure.
FIG. 4 is a schematic block diagram of a controller used in an oral care system illustrated according to aspects of the present disclosure.
FIG. 5 is a diagram of an oral condition detection module and associated oral care device illustrated according to aspects of the present disclosure.
FIG. 6 is a flowchart of a method for detecting an oral condition illustrated according to aspects of the present disclosure.
FIG. 7 is a drawing of an oral treatment procedure being performed using an oral care device illustrated according to aspects of the present disclosure.

### Detailed Description of Embodiments

The present disclosure is directed to systems and methods for detecting an oral condition of a subject using an oral care device, for example during an oral treatment procedure. More specifically, the present disclosure is directed to systems and methods that employ an oral care device configured to induce thermal changes over a target region within a subject's oral cavity, and then thermally detect one or more oral conditions. According to preferred embodiments, the inducement and detection may be performed using the oral care device during an oral treatment procedure (i.e., while simultaneously performing the oral treatment procedure). As described herein, Applicants have understood and appreciated that the different organic and/or inorganic matter within the oral cavity may have different thermal properties and therefore produce differing temperature gradients that can be distinguished in order to provide information on a number of potential oral conditions.

In some embodiments, the systems and methods of the present disclosure may be used without applying any dyes, special formulations, contrast agents, and/or the like. In some embodiments, the systems and methods of the present disclosure may be used alone or in conjunction with one or more external devices. In some embodiments, the systems and methods may be used while the oral treatment procedure is ongoing (e.g., while toothpaste slurry is still in the mouth, etc.). In some embodiments, the systems and methods of the present disclosure may be used to provide feedback to a user or operator, for example, while the treatment procedure is still ongoing.

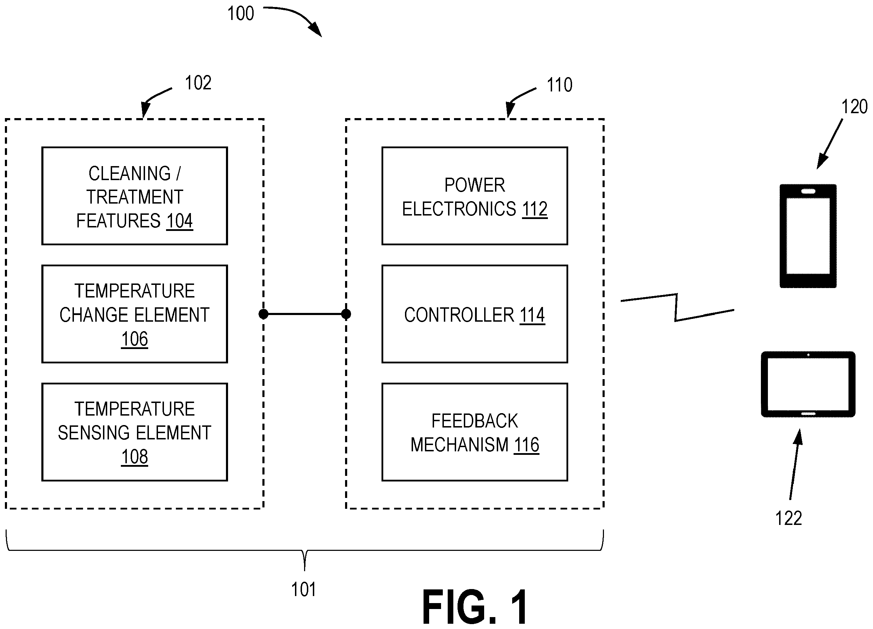

Turning to FIG. 1, an oral care system 100 comprising an oral care device 101 is illustrated according to aspects of the present disclosure. In embodiments, the oral care device 101 comprises a head portion 102 adapted and/or configured to be received in an oral cavity of a subject and to be used for an oral treatment procedure. In some embodiments, the head portion 102 of the oral care device 101 can include one or more treatment features 104, a temperature change element 106, and/or a temperature detector 108.

In embodiments, the treatment features 104 can be, but are not limited to, elements, structures, and/or combinations thereof adapted to facilitate the oral treatment procedure being performed on the subject. For example, in some embodiments, the oral care device 101 is an electric toothbrush and the treatment features 104 comprise bristles, silicone pillars, woven textiles, and/or combinations thereof. In further embodiments, the oral care device 101 can be a dental polishing device and the treatment features 104 comprise a polishing cup, a flat top brush, a tip brush, a polishing bur, and/or the like.

In still further embodiments, the oral care device 101 may be a surgical tool used in oral surgery. For example, the surgical tool may include a cutting instrument, a drilling instrument, and the like. In such embodiments, the oral care device 101 can be configured to detect the type and/or condition of tissue the surgeon is operating on, as well as distinguish between different types and/or conditions of tissues.

In particular embodiments, the temperature change element 106 is configured to induce a change in temperature of at least a target region of an oral cavity when the head portion 102 of the oral care device 101 is received in said oral cavity. In embodiments, the temperature change element 106 can induce changes in temperature while the oral treatment procedure is being performed (e.g., while a user is brushing their teeth, while a dental hygienist is polishing a subject's teeth, while an oral surgeon is performing an operation, etc.).

In some embodiments, the temperature change element 106 can comprise one or more nozzles and/or apertures, as well as a pump, a fan, and/or the like configured to produce an airstream that is directed to the target region of the subject's oral cavity via the one or more nozzles or apertures. Put another way, the temperature change element 106 can be configured to induce a change in temperature within the target region of the oral cavity during the oral treatment procedure by directing an airstream to the target region of the oral cavity. As a result of the airstream produced by the temperature change element 106, evaporative cooling of the target region of the oral cavity may be achieved in some embodiments. In further embodiments, the temperature change element 106 may include a heating element, such as a resistive heating element, configured to warm the airstream before exiting the one or more nozzles or apertures. As a result, heating of the target region may also be achieved in some embodiments.

In further embodiments, the temperature change element 106 can comprise one or more nozzles and/or apertures, as well as a pump and/or the like configured to produce a liquid stream that is directed to the target region of the subject's oral cavity via the one or more nozzles or apertures. Put another way, the temperature change element 106 can be configured to induce a change in temperature within the target region of the oral cavity during the oral treatment procedure by directing a liquid stream to the target region of the oral cavity. As a result of the liquid stream produced by the temperature change element 106, cooling of the target region of the oral cavity may be achieved in some embodiments. In further embodiments, the temperature change element 106 may include a heating element, such as a resistive heating element, configured to warm the liquid stream before exiting the one or more nozzles or apertures. As a result, heating of the target region may also be achieved in some embodiments.

In still further embodiments, the temperature change element 106 can comprise an electromagnetic (EM) radiation source configured to emit EM radiation having one or a range of wavelengths that is directed to the target region of the subject's oral cavity via one or more nozzles or apertures. In embodiments, the EM radiation source may produce, for example and without limitation, high frequency radio waves, microwaves, infrared waves, ultraviolet waves, and/or visible light.

Accordingly, the temperature change element 106 can be configured to induce a change in temperature within the target region of the oral cavity during the oral treatment procedure by directing EM radiation to the target region of the oral cavity. As a result of the EM radiation produced by the temperature change element 106, heating of the target region of the oral cavity may be achieved in some embodiments.

In yet further embodiments, the temperature change element 106 can comprise a heated and/or cooled element configured to contact at least a portion of the target region. For example, in embodiments, the temperature change element 106 may include one or more heated bristles and/or pillars. In other embodiments, the temperature change element 106 may include one or more cooled bristles and/or pillars. Accordingly, by contacting the temperature change element 106 with at least a portion of the target region, a heat exchange may be achieved to induce a change in temperature.

In accordance with the present disclosure, the temperature sensing element 108 is configured to detect or otherwise measure the variations in temperature over at least the target region of the oral cavity, including variations in temperature produced by the temperature change element 106, when the head portion 102 of the oral care device 101 is received in said oral cavity. In embodiments, the temperature sensing element 108 can detect variations in temperature while the oral treatment procedure is being performed (e.g., while a user is brushing their teeth, while a dental hygienist is polishing a subject's teeth, while an oral surgeon is performing an operation, etc.).

In embodiments, the temperature sensing element 108 is configured to detect or otherwise measure the variations in temperature over at least the target region of the oral cavity, including variations in temperature produced by the temperature change element 106. In preferred embodiments, the variations in temperature are temperature gradients of different organic and/or inorganic material within the oral cavity. Preferably, the temperature gradients are produced alone or in part by the temperature change element 106. For example, the temperature change element 106 may use an airstream, a liquid stream, EM radiation, or another suitable method to cause heating and/or cooling of the matter at the target region of the subject's oral cavity. As a result of applying an airstream, liquid stream, EM radiation, and/or the like to the target region, different temperature gradients can be produced in which certain matter in the oral cavity heats and/or cools at different rates and to different degrees than other nearby organic and/or inorganic matter. In embodiments, these differences in temperature gradients can be due to, for example and without limitation, surface deposits and defects such as plaque and cavities, respectively. As described herein, the temperature sensing element 108 can be used to detect temperature variations produced when such organic and/or inorganic matter within the oral cavity is heated and/or cooled by a temperature change element 106.

In some embodiments, the temperature sensing element 108 can include a thermal camera. For example, and without limitation, the temperature sensing element may comprise an infrared detector to detect infrared energy that is provided to the infrared detector through an infrared camera lens.

In further embodiments, the temperature sensing element 108 can include a temperature sensor and/or an element of temperature sensors. For example, and without limitation, the temperature sensing element 108 can include one or more thermal radiation sensors, thermocouples, resistance temperature detectors, thermistors, and/or the like.

In still further embodiments, the temperature sensing element 108 can include one or more contact temperature sensors that are configured to contact one or more parts of the oral cavity within the target region. For example, and without limitation, the temperature sensing element 108 can include one or more contact temperature sensors that are thermocouples, resistance temperature detectors, thermowells, and/or the like.

In accordance with the present disclosure, the target region of a subject's oral cavity can be, for example and without limitation, one or more teeth, a portion of the gumline, the soft palate or a portion thereof, the hard palate or a portion thereof, the floor of the mouth, the buccal mucosa or a portion thereof, and/or combinations thereof. In embodiments, the oral care device 101 may be moved around, rotated, and/or otherwise manipulated within the oral cavity of the subject in order to provide multiple different target regions of the oral cavity. That is, for example, while the oral care device 101 is used to perform the treatment procedure, the oral care device 101 may be manipulated within the subject's oral cavity and thereby provide multiple different target regions over time.

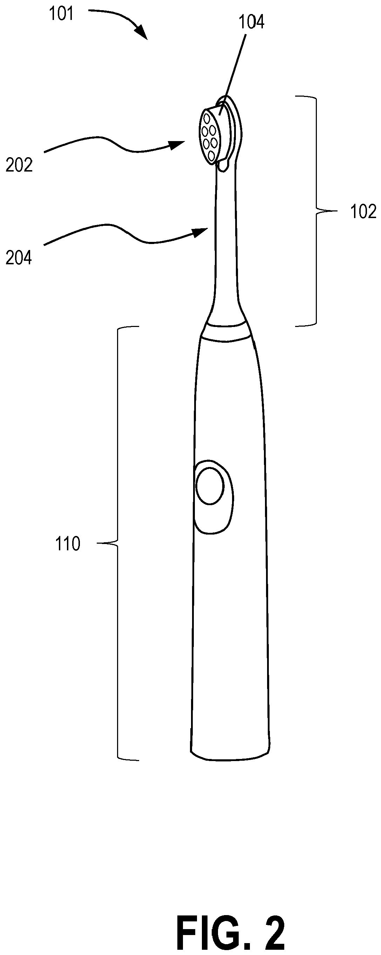

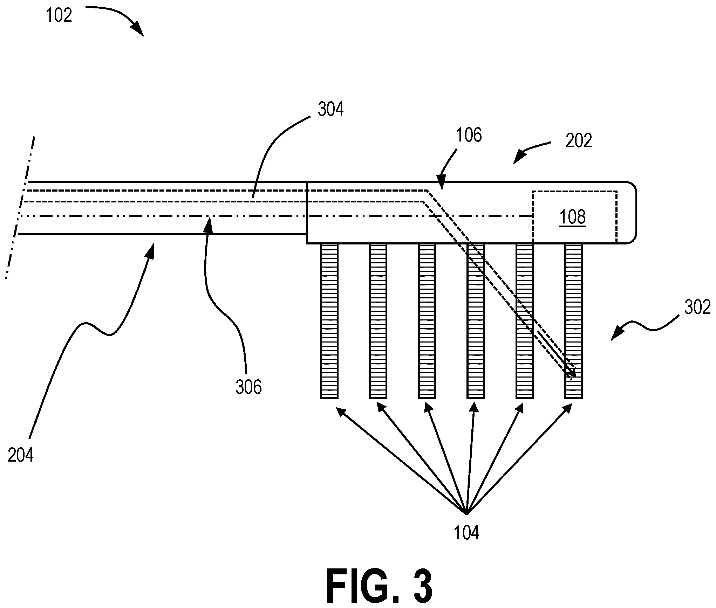

With reference to FIG. 2 and FIG. 3, an oral care device 101 is illustrated according to certain embodiments of the present disclosure. As shown in FIG. 2, the oral care device 101 includes a head portion 102 and a body portion 110. The head portion 102 can include a first end 202 where the treatment features 104 are disposed, and a stem (also referred to as a neck) 204 that operatively connects the head portion 102 to the body portion 110. As shown in FIG. 3, the head portion 102 can include the temperature change element 106 and the temperature sensing element 108. In particular, as shown, the temperature change-inducing stream 302 (e.g., an airstream, liquid stream, EM radiation, etc.) is directed out of the head portion 102 and towards a target region of the subject's oral cavity, while the temperature sensing element 108 is positioned (either internally and/or externally on the head portion 102) such that temperature variations caused by the temperature change element 106 within the target region can be detected.

In embodiments, the components of the temperature change element 106 and the temperature sensing element 108 may be concentrated in the head portion 102, or may be distributed between the head portion 102 and the body portion 110. For example, in some embodiments, the neck 204 of the head portion 102 may operatively connect one or more components of the temperature change element 106 and/or the temperature sensing element 108 disposed at or within the head portion 102 with one or more components at or within the body portion 110. As shown, for example, the neck 204 and the first end 202 of the head portion 102 can include a conduit 304 for delivering an airstream, liquid stream, and/or EM radiation from the body portion 110 through the head portion 102 and to the target region of the subject's oral cavity.

Accordingly, in some embodiments, the body portion 110 can include one or more components of either the temperature change element 106 and/or the temperature sensing element 110. In further embodiments, as shown in FIG. 1, the body portion 110 can also include at least one of power electronics 112, a controller or microcontroller 114, and/or a feedback mechanism 116. In particular embodiments, the body portion 110 is configured to be handheld by a single user.

In embodiments, the power electronics 112 comprise one or more power sources, such as rechargeable power sources, and be configured to provide power to at least the temperature change element 106 and the temperature sensing element 108. In some embodiments, the power electronics 112 further provide power to the treatment features 104 and/or components associated with said features 104 in order to effectuate the treatment procedure.

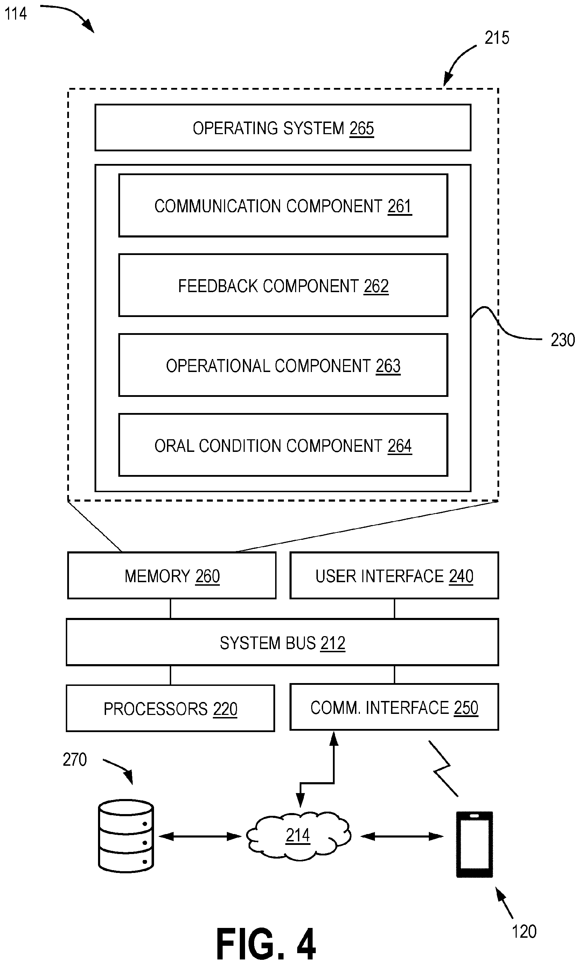

In embodiments, the controller 114 can be configured to operate at least the temperature change element 106 and the temperature sensing element 108, as well as the treatment features 104. With reference to FIG. 4, an example controller 114 for an oral care device 101 is illustrated.

In embodiments, the controller 114 comprises one or more processors 220, machine-readable memory 260, a user interface 240, and/or a communications interface 250, all of which may be interconnected and/or communication through a system bus 212 containing conductive circuit pathways through which instructions (e.g., machine-readable signals) may travel to effectuate communication, tasks, storage, and the like.

As discussed in more detail below, the one or more processors 220 may be configured to perform one or more steps of the methods described herein, including but not limited to, the following: (i) inducing a change in temperature of a target region of the oral cavity using the temperature change element 106 of the oral care device 101; (ii) detecting variations in temperature over at least the target region of the oral cavity using the temperature sensing element 106 of the oral care device 101; (iii) determining, based on the detected variations in temperature over at least the target region of the oral cavity, one or more oral conditions; and/or (iv) providing, via a feedback mechanism 116, an alert to a user of the oral care device 101 indicating the existence of the one or more oral condition within the oral cavity of the subject.

In some embodiments, the one or more processors 220 may include a high-speed data processor adequate to execute the program components described herein and/or various specialized processing units as may be known in the art. In some examples, the one or more processors 220 may be a single processor, multiple processors, or multiple processor cores on a single die.

In some embodiments, the communications interface 250 can include a network interface configured to connect the controller 114 to a communications network 214, an input/output ("I/O") interface configured to connect and communicate with one or more peripheral devices, a memory interface configured to accept, communication, and/or connect to a number of machine-readable memory devices, and the like.

In certain embodiments, the communications interface 250 may operatively connect the controller 114 to a communications network 214, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In some examples, ICU LOS prediction system 200 may communicate with one or more remote / cloud-based servers (e.g., the electronic medical records database 270), cloud-based services, and/or remote devices via the communications network 214.

The memory 260 can be variously embodied in one or more forms of machine-accessible and machine-readable memory. In some examples, the memory 260 includes a storage device that comprises one or more types of memory. For example, a storage device can include, but is not limited to, a non-transitory storage medium, a magnetic disk storage, an optical disk storage, an array of storage devices, a solid-state memory device, and the like, including combinations thereof.

Generally, the memory 260 is configured to store data / information and instructions 215 that, when executed by the one or more processors 220, causes the controller 114 to perform one or more tasks. In particular examples, the memory 260 includes thermal sensing package 230 that causes the controller 114 to perform one or more steps of the methods described herein.

In embodiments, the thermal sensing package 230 comprises a collection of program components, database components, and/or data. Depending on the particular implementation, the thermal sensing package 230 may include software components, hardware components, and/or some combination of both hardware and software components.

The thermal sensing package 230 may include one or more software packages configured to: operate the temperature change element to induce a change in temperature of a target region of the oral cavity; operate the temperature sensing element to detect variations in temperature over at least the target region of the oral cavity; determine, based on the detected variations in temperature over at least the target region of the oral cavity, one or more oral conditions; and/or generate an alert indicating the one or more oral conditions. In further embodiments, the thermal sensing package 230 may include one or more software packages configured to communicate with an external device (e.g., devices 120, 122) that may determine, based on the detected variations in temperature over at least the target region of the oral cavity, one or more oral conditions; and/or generate an alert indicating the one or more oral conditions. These software packages may be incorporated into, loaded from, loaded onto, or otherwise operatively available to and from the controller 114.

In some examples, the thermal sensing package 230 and/or one or more individual software packages may be stored in a local storage device 260. In other examples, the thermal sensing package 230 and/or one or more individual software packages may be loaded onto and/or updated from a remote server via the communications interface 250.

In particular embodiments, the thermal sensing package 230 can include, but is not limited to, instructions 215 having a communication component 261, a feedback component 262, an operational component 263, and/or an oral condition component 264. These components may be incorporated into, loaded from, loaded onto, or otherwise operatively available to and from the controller 114.

In embodiments, the communication component 261 can be a stored program component that is executed by at least one processor, such as the one or more processors 220 of the controller 114. In particular, the communication component 261 can be configured to interface with an electronic medical records database 270 and/or one or more external device 120 in order to transmit and/or receive temperature variation information from the oral care device 101.

In embodiments, the feedback component 262 can be a stored program component that is executed by at least one processor, such as the one or more processors 220 of the controller 114. In particular, the feedback component 262 can be configured to generate an alert indicating the one or more oral conditions, if detected. For example, in embodiments, the alert may be delivered at least via a feedback mechanism 116 of the oral care device 101. In some embodiments, the feedback mechanism 116 may include a vibrating element configured to produce a vibration from within the oral care device 101, and/or may include an audio-producing element configured to produce an audible alert from within the oral care device 101.

In embodiments, the operational component 263 can be a stored program component that is executed by at least one processor, such as the one or more processors 220 of the controller 114. In particular, the operational component 263 can be configured to: operate the temperature change element 106 to induce a change in temperature of a target region of the oral cavity; operate the temperature sensing element 108 to detect variations in temperature over at least the target region of the oral cavity; and/or operate the cleaning or treatment features 104 to effectuate the treatment procedure.

In embodiments, the oral condition component 264 can be a stored program component that is executed by at least one processor, such as the one or more processors 220 of the controller 114. In particular, the oral condition component 264 can be configured to analyze the temperature variation information and determine, based on the temperature variation information, whether one or more oral conditions exist within at least the target region of the subject's oral cavity.

In some embodiments, the controller 114 may be at least part of a larger system 100, which may also include an electronic medical records database 270 and/or one or more external devices 120, 122. In embodiments, the one or more external devices 120, 122 may facilitate or supplement one or more steps of the methods described herein. For example, in particular embodiments, feedback alerts may be presented to the user of the oral care device 101 via the one or more external devices 120, 122 in lieu of or in addition to the feedback mechanism 116 of the oral care device 101.

The controller 114 may also include an operating system component 265, which may be stored in the memory 260. The operating system component 265 may be an executable program facilitating the operation of the controller 114. Typically, the operating system component 265 can facilitate access of the communications interface 250, and can communicate with other components of the controller 114, including but not limited to, the user interface 240, the memory 260, and/or the electronic medical records database 270.

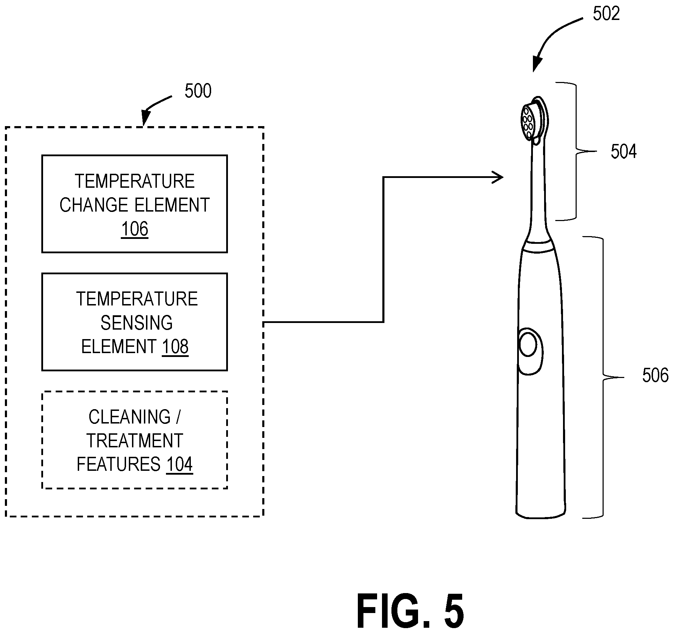

Also provided herein are oral condition detection modules 500 configured to be attached to an existing oral care device (e.g., a toothbrush, an electric toothbrush, and/or the like). For example, with reference to FIG. 5, an oral condition detection module 500 configured to be attached to an existing oral care device 502, wherein the oral condition detection module 500 includes at least a temperature change element 106 and a temperature sensing element 108 as described above. The oral condition detection module 500 can include a flexible and/or rigid housing that secures the temperature change element 106 and/or temperature sensing element 108 to the oral care device 502. In particular embodiments, the oral condition detection module 500 may be a click-on or snap-on module 500 that fits onto an existing device and/or tool, such as an existing toothbrush. In embodiments, the oral condition detection module 500 can include a power source and/or power electronics configured to receive power from an external power source.

In preferred embodiments, the oral condition detection module 500 is adapted to be received in a subject's oral cavity. Put another way, the oral condition detection module 500 may be configured to be attached to a head portion 504 of the oral care device 504 such that the head portion 504 and the oral condition detection module 500 are concurrently received in the subject's oral cavity.

In other preferred embodiments, the oral condition detection module 500 is a detachable head portion 504 of an oral care device 502. For example, the oral condition detection module 500 may optionally include one or more treatment features 104 and be configured to reversibly attach to a body portion 506 of an oral care device 502.

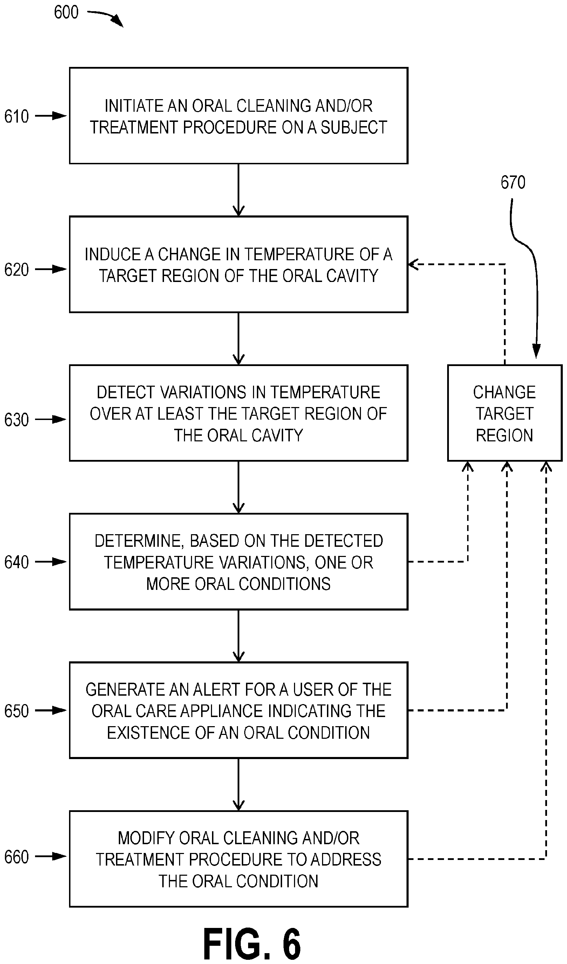

Also provided herein are methods of detecting an oral condition of a subject. With reference to FIG. 6, the method 600 can include: in a step 610, initiating an oral treatment procedure on the subject; in a step 620, inducing a change in temperature of a target region of the subject's oral cavity; in a step 630, detecting variations in temperature over at least the target region of the subject's oral cavity; and in a step 640, determining one or more oral conditions based on the detected temperature variations. In embodiments, the method 600 can also include: in a step 650, generating an alert for a user of the oral care device 101 indicating the one or more oral conditions. In some embodiments, the method 600 can include: in a step 660, modifying the oral treatment procedure in response to the one or more oral conditions.

More specifically, in a step 610, the method 600 can include initiating an oral treatment procedure on the subject utilizing an oral care system 100 comprising an oral care device 101. According to the present disclosure, the oral treatment procedure can be an at-home toothbrushing, a dental cleaning by a dental hygienist, an oral surgery operation, and/or the like. As discussed herein, the oral care device 101 can include (i) a head portion 102 adapted to be received in the oral cavity of the subject for the oral treatment procedure, and (ii) a body portion 110 operatively connected to the head portion 102. In particular embodiments, the body portion 110 is configured to be handheld by a single user.

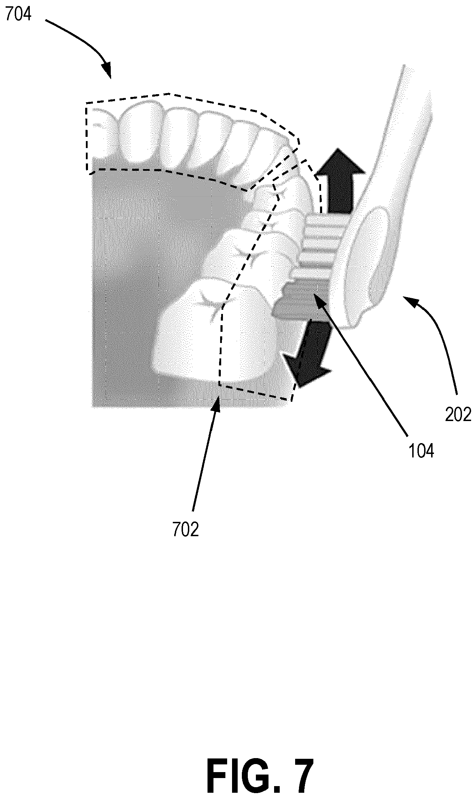

For example, with reference to FIG. 7, an at-home toothbrushing event is illustrated. As shown, the first end 202 of the head portion 102 comprises a plurality of cleaning features 104 that contact the teeth and gums of the subject.

In a step 620, the method 600 can include inducing a change in temperature of a target region of the oral cavity of the subject using a temperature change element 106 of the oral care device 101. In embodiments, the target region of a subject's oral cavity can be, for example and without limitation, one or more teeth, a portion of the gumline, the soft palate or a portion thereof, the hard palate or a portion thereof, the floor of the mouth, the buccal mucosa or a portion thereof, and/or combinations thereof. As shown in FIG. 7, the cleaning elements 104 of the oral care device 101 contact the teeth and gums within the target region 702 including a front-facing section of the subject's oral cavity.

As discussed herein, the temperature change element 106 may include one or more components configured to direct an airstream, a liquid stream, and/or EM radiation towards the target region 702 of the subject's oral cavity. In some embodiments, the changes in temperature produced by the temperature change element 106 may include a heating effect and/or a cooling effect (i.e., the target region 702 may be warmed to an elevated temperature and/or cooled to a lower temperature). In further embodiments, inducing a change in temperature of the target region of the oral cavity of the subject may be effectuated using the temperature change element 106 in less than about 2 seconds, including less than about 1 second, and less than about 0.5 seconds.

In a step 630, the method 600 can include detecting variations in temperature over at least the target region 702 of the oral cavity using a temperature sensing element 108 of the oral care device 101. More specifically, the step 630 includes detecting variations in temperature caused by the temperature change element 106 within the target region 702. In embodiments, the temperature sensing element 108 can include a thermal camera, temperature sensor and/or an element of temperature sensors, and/or one or more contact temperature sensors, as discussed herein. In further embodiments, the detecting and/or measuring of the variations in temperature over at least the target region 702 of the oral cavity may be performed within about 2 seconds, including within about 1 second and within about 0.5 seconds.

In a step 640, the method 600 can include determining, based on the detected variations in temperature, one or more oral conditions. In embodiments, the one or more oral conditions can include at least one of the presence of dental plaque, dental caries, dental calculus, tooth decay, enamel defects, cavities, and/or soft tissue infection. In further embodiments, determining the one or more oral conditions also includes determining a degree of severity or otherwise classifying the type of the oral condition. In some embodiments, the one or more oral conditions may be determined based on the temperature variations detected over multiple different target regions 702, 704, or may be determined based on a single target region 702, 704. For example, in some embodiments, the step 640 includes determining the presence and/or amount of plaque on the subject's teeth within a single target region 702, 704.

In some embodiments, the step 640 may be performed by a controller or microcontroller 114 of the oral care device 101, or may be performed by an external device 120, 122 such as a mobile phone, tablet, or other personal computing device.

In a step 650, the method 600 can include generating an alert for a user of the oral care device 101 indicating the one or more oral conditions. In some embodiments, the alert may be generated using a feedback mechanism 116 within the oral care device 101, and/or generated using an external device 120, 122. In some embodiments, a first alert may be generated using the feedback mechanism 116 within the oral care device 101 while a second alert is generated using the external device 120, 122, such that the first alert is a simple vibration or audible alert, while the second alert contains additional details about the detected oral condition(s).

In a step 660, the method 600 can include modifying the oral treatment procedure based on the generated alert. In particular embodiments, in some embodiments, the oral treatment procedure may be modified to address the identified oral condition(s). For example, in some embodiments, the presence of a significant amount of plaque may be detected within the target region 702 such that the operator of the oral care device 101 is notified in accordance with the present disclosure, at which point the operator of the oral care device 101 may return to the target region 702 and/or spend additional time cleaning the target region 702.

In particular embodiments, the method 500 can also include, in a step 670, changing the target region 702, 704 and repeating the steps 620, 630, 640, 550, and/or 660 any number of times. For example, as discussed herein, the oral care device 101 may be moved around, rotated, and/or otherwise manipulated within the oral cavity of the subject in order to provide multiple different target regions 702, 704 of the oral cavity over time.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An oral care system (100) comprising:
an oral care device (101) including (i) a head portion (102) adapted to be received in an oral cavity of a subject for an oral treatment procedure, and (ii) a body portion (110) operatively connected to the head portion (102);
wherein the head portion (102) of the oral care device (101) comprises:
a temperature change element (106) configured to induce a change in temperature of a target region of the oral cavity; and
a temperature sensing element (108) configured to detect variations in temperature over at least a section of the target region of the oral cavity.

2. The oral care system (100) of claim 1, wherein the head portion (102) of the oral care device (101) further comprises a treatment feature (104) configured to facilitate the oral treatment procedure, and the temperature change element (106) is configured to induce a change in temperature of a target region of the oral cavity during the oral treatment procedure in proximity thereof.

3. The oral care system (100) of claim 2, wherein the treatment feature (104) comprises at least one of bristles, pillars, and woven textiles.

4. The oral care system (100) of claim 2, wherein the temperature change element (106) is configured to induce a change in temperature of the target region of the oral cavity during the oral treatment procedure by at least one of: (i) directing an airstream to the target region of the subject's oral cavity; (ii) directing a liquid stream to the target region of the subject's oral cavity; (iii) directing electromagnetic radiation to the target region of the subject's oral cavity; and (iv) heating and/or cooling the temperature change element and then contacting at least a portion of the target region with the temperature change element.

5. The oral care system (100) of claim 1, wherein the oral care system (100) is configured to determine an oral condition within the oral cavity of the subject based on the detected variations in temperature induced by the temperature change element (106) over at least the target region of the oral cavity.

6. The oral care system (100) of claim 5, wherein the oral condition is at least one of the presence of dental plaque, dental caries, dental calculus, tooth decay, enamel defects, cavities, and/or soft tissue infection.

7. The oral care system (100) of claim 1, wherein the temperature sensing element (108) includes at least one of a thermal camera, a thermal radiation sensor or sensor array, and/or a contact temperature sensor.

8. The oral care system (100) of claim 1, wherein the body portion (110) of the oral care device comprises a user feedback mechanism (116) configured to provide an alert indicating the existence of an oral condition within the oral cavity of the subject.

9. The oral care system (100) of claim 1, further comprising one or more processors (220) in communication with the oral care device (101) and configured to:
operate the temperature change element (106) to induce a change in temperature of a target region of the oral cavity;
operate the temperature sensing element (108) to detect variations in temperature over at least a section of the target region of the oral cavity;
determine, based on the detected variations in temperature over at least the target region of the oral cavity, one or more oral conditions; and
generate an alert indicating the one or more oral conditions.

10. A method (600) of detecting an oral condition of a subject, the method comprising:
initiating (610) an oral treatment procedure on the subject utilizing an oral care system comprising an oral care device, the oral care device including (i) a head portion adapted to be received in an oral cavity of the subject for the oral treatment procedure, and (ii) a body portion operatively connected to the head portion, wherein the head portion of the oral care device comprises (i) a temperature change element configured to induce a change in temperature of a target region of the oral cavity during the oral treatment procedure, and (ii) a temperature sensing element configured to detect variations in temperature over at least a section of the target region of the oral cavity;
while performing the oral treatment procedure:
inducing (620) a change in temperature of a target region of the oral cavity using the temperature change element of the oral care device; and
detecting (630) variations in temperature over at least the section of the target region of the oral cavity using the temperature sensing element of the oral care device.

11. The method (600) of claim 10, wherein the body portion of the oral care device comprises a user feedback mechanism configured to provide an alert indicating the existence of an oral condition within the oral cavity of the subject, and wherein the method further comprises:
determining (640), based on the detected variations in temperature over at least the target region of the oral cavity, one or more oral conditions; and
providing (650), via the feedback mechanism, an alert to a user of the oral care device indicating the existence of the one or more oral condition within the oral cavity of the subject.

12. The method (600) of claim 10, wherein the oral condition is at least one of the presence of dental plaque, dental caries, dental calculus, tooth decay, enamel defects, cavities, and/or soft tissue infection.

13. An oral condition detection module (500) configured to be attached to an oral care device (502), the oral condition detection module (500) comprising:
a temperature change element (106) configured to induce a change in temperature of a target region of a subject's oral cavity; and
a temperature sensing element (108) configured to detect variations in temperature over at least a section of the target region of the subject's oral cavity;
wherein the oral condition detection module is configured to be attached to a head portion (504) of the oral care device (502), the head portion (504) and the oral condition detection module (500) being configured to be received in the subject's oral cavity.

14. The oral condition detection module (500) of claim 13, wherein the temperature change element (106) is configured to induce a change in temperature of the target region of the subject's oral cavity during the oral treatment procedure by at least one of: (i) directing an airstream to the target region of the subject's oral cavity; (ii) directing a liquid stream to the target region of the subject's oral cavity; (iii) directing electromagnetic radiation to the target region of the subject's oral cavity; and (iv) heating and/or cooling the temperature change element and then contacting at least a portion of the target region with the temperature change element.

15. The oral condition detection module (500) of claim 13, further comprising a user feedback mechanism (116) configured to provide an alert indicating the existence of an oral condition within the subject's oral cavity.
